(19) Europäisches Patentamt
European Patent Office
Office européen des brevets

(11) Publication number: **0 052 437**
**A1**

(12) # EUROPEAN PATENT APPLICATION

(21) Application number: 81304988.9

(22) Date of filing: 23.10.81

(51) Int. Cl.³: C 07 D 407/06
C 07 D 309/10, A 61 K 31/35

(30) Priority: 12.11.80 GB 8036256
13.08.81 GB 8124818

(43) Date of publication of application:
26.05.82 Bulletin 82/21

(84) Designated Contracting States:
BE CH DE FR IT LI NL

(71) Applicant: BEECHAM GROUP PLC
Beecham House Great West Road
Brentford Middlesex(GB)

(72) Inventor: O'Hanlon, Peter John
65 Green Lane
Redhill Surrey(GB)

(72) Inventor: Walker, Graham
66 Agraria Road
Guildford Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

(54) **Antibacterial compounds, processes for their preparation and compositions containing them.**

(57) Compounds of formula (II):

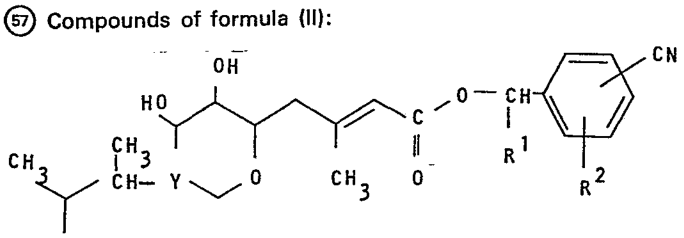

(11)

wherein Y is selected from $-CH=CH-CH_2-CH-$,

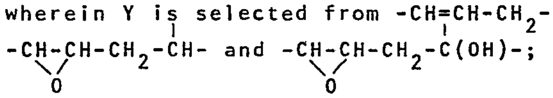

and ;

$R^1$ represents hydrogen or a $C_{1-4}$ alkyl group and $R^2$ is hydrogen or a cyano group;
have activity against human and veterinary bacteria and mycoplasma.

EP 0 052 437 A1

## Antibacterial Compounds, Processes For Their Preparation and Compositions Containing Them

This invention relates to antibacterial compounds and in particular to a class of esters which have antibacterial activity against certain Gram-positive and Gram-negative organisms, and also possess anti-mycoplasmal activity. The compounds are therefore of value in the treatment of human and veterinary infections.

The compounds of formula (I) and salts and esters thereof:

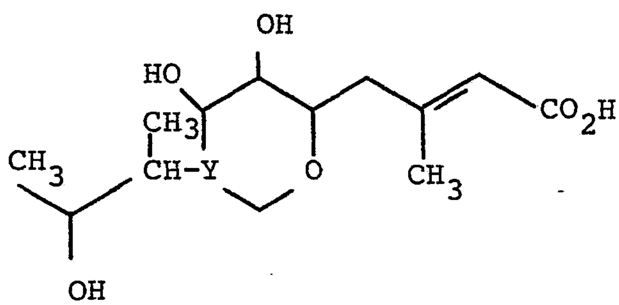

(I)

wherein Y represents $-CH=CH-CH_2-\overset{|}{C}H-$, 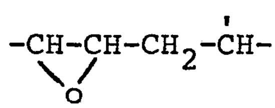

and 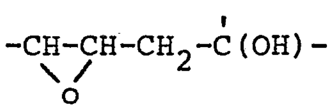 are disclosed in West Germany Offenlegungsschriften No. 2726619, 2726618 and 2848687 and European Patent Application No. 79300371.6. Compounds of formula (I) having the tri-substituted double bond in the E-configuration are referred to as monic acid C, monic acid A and monic acid B respectively.

We have now found a small group of cyano esters of the compounds of formula (I) which have particularly good antibacterial and antimycoplasmal activity.

Accordingly, the present invention provides an ester of formula (II):

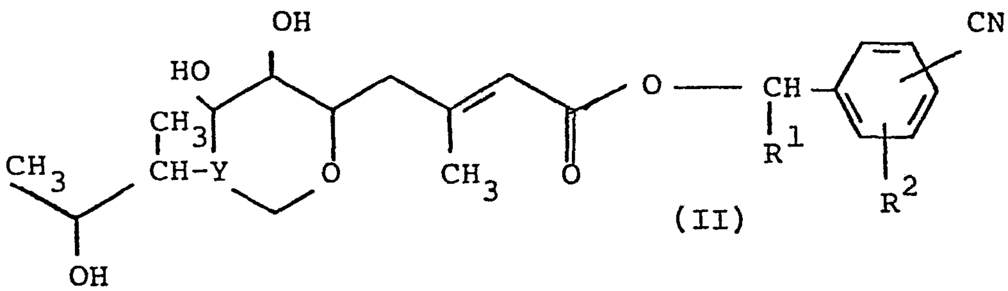

(II)

wherein Y is as defined with respect to formula (I); and $R^1$ represents hydrogen or a $C_{1-4}$ alkyl group and $R^2$ is hydrogen or cyano. Preferably the compounds of formula (II) are derivatives of monic acid A or C, i.e. Y represents

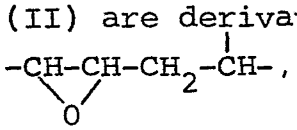 , or 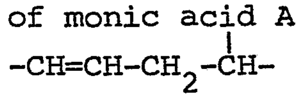

Suitably, $R^1$ represents a hydrogen atom or a methyl group. When $R^2$ is hydrogen the cyano group may be in any position on the aromatic ring, but a preferred position is the m-position. When $R^2$ is cyano the cyano groups may be in any position on the aromatic ring but preferably they are in the two m-positions.

The compounds (II) of this invention incorporate a tri-substituted double bond and may therefore exist in both the E (natural) and Z (or iso) geometrical forms. It is to be understood that both geometrical isomers of the compounds of formula (II) are included within the scope of this invention, as well as mixtures of the two isomers. However, because in general the E-isomer of a particular derivative of compound (II) has the greater activity, it is preferable to employ that isomer.

In addition the 10, 11 double bond in the C-series of compounds is naturally E in configuration.

Compounds of this invention have antibacterial and antimycoplasmal activity, and are therefore of value in the treatment of bacterial and mycoplasma-induced human and veterinary diseases.

The infections against which compounds of this invention are particularly useful include venereal disease. They are also effective in the treatment of respiratory infections such as bacterial bronchitis; and bacterial meningitis, non-specific urethritis and pneumonia. In animals it may be employed for the treatment of mastitis in cattle, for swine dysentery, and for mycoplasma infections in animals such as turkeys, chickens, pigs and cattle.

Some of the human and veterinary diseases either caused by mycoplasma species or in which they play a prominent role, and against which compounds of this invention are effective, are as follows:

Avian

| | | |
|---|---|---|
| M gallisepticum | - | Chronic respiratory diseases (air-sacculitis) of chickens and turkeys |

Bovine

| | | |
|---|---|---|
| M-bovis | - | Mastitis, respiratory disease and arthritis of cattle |
| M dispar | - | Calf pneumonia |

Porcine

M suipneumoniae - Enzootic pneumonia of pigs

M hyorhinis )
M hyosynoviae ) - arthritis in pigs

Human

M pneumoniae - primary atypical pneumonia

Compounds of the present invention are particularly useful in the treatment of enzootic pneumonia in animals such as pigs, cattle and sheep, because they also have activity against the bacteria Bordetella bronchispetica, Pasteurella multocida and Haemophilus spp, which often cause respiratory complications in cases of this disease.

This invention also provides a pharmaceutical or veterinary composition which comprises a compound of formula (II) together with a pharmaceutically or veterinary acceptable carrier or excipient.

The compositions may be formulated for administration by any route, and would depend on the disease being treated. The compositions may be in the form of tablets, capsules, powders, granules, lozenges, liquid or gel preparations, such as oral, topical or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinyl-pyrollidone; fillers, for example lactose, sugar, maize-starch, calcium phosphate, sorbitol or

glycine; tabletting lubricants, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin compounds of this invention may be made up into a cream, lotion or ointment. Cream or ointment formulations that may be used for compounds of formula (II) are conventional formulations well known in the art, for example, as described in standard text books of pharmaceutics and cosmetics, such as Harry's Cosmeticology published by Leonard Hill Books, and the British Pharmacopoeia.

Suppositories will contain conventional suppository bases, eg cocoa-butters or other glyceride.

For parenteral administration, fluid unit dosage forms are prepared utilizing the compound and a sterile vehicle, water being preferred. The compound, depending on the vehicle and concentration used, can either be suspended or dissolved in the vehicle. In preparing solutions the compound can be dissolved in water for injection and filter sterilized before filling into a suitable vial or ampoule and sealing. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compounds.

Veterinary compositions for intramammary treatment of mammary disorders in animals, especially bovine mastitis, will generally contain a suspension of a compound of formula (II) in an oily vehicle.

The compositions may contain from 0.1% to 99% by weight, preferably from 10-60% by weight, of the active material, depending on the method of administration. Where the compositions comprise dosage units, each unit will preferably contain from 50-500 mg, of the active ingredient. The dosage as employed for adult human

treatment will preferably range from 100 mg to 3 g, per day, for instance 250 mg to 2 g, per day, depending on the route and frequency of administration.

Alternatively a compound of formula (II) may be administered as part of the total dietary intake. In this case the amount of compound employed may be less than 1% by weight of the diet and in preferably no more than 0.5% by weight. The diet for animals may consist of normal foodstuffs to which the compound may be added or it may be added to a premix.

A suitable method of administration of a compound of formula (II) to animals is to add it to the animals' drinking water. In this case a concentration of compound in the drinking water of about 5-500 μg/ml, for example 5-200 μg/ml, is suitable.

Compounds of formula (II) may be prepared by reacting a cyano-benzyl compound of formula (III):

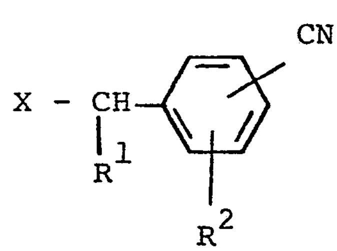

(III)

in which X is a good leaving group, preferably a halogen atom or an alkyl or aryl sulphonate, and $R^1$ and $R^2$ are as defined in formula (II) with a salt of the acid of formula (I), preferably the sodium salt. Preferably, X is a bromine atom or a mesylate group.

The reaction is preferably carried out in an organic solvent, suitably dimethylformamide, at 20° to 100°C

and the resulting product may be purified chromatographically on silica.

Compounds of formula (II) wherein Y represents $-CH=CH-CH_2-\overset{|}{C}H-$ may also be prepared from compounds of formula (II) wherein Y represents $-\underset{\diagdown O \diagup}{CH-CH}-CH_2-\overset{|}{C}H-$ by reaction with a reagent which converts an epoxide to an olefin. Such reactions are described in our European Patent Applications Nos. 78300530.9 and 79300371.6.

The following Examples illustrate the preparation of various compounds of the present invention.

EXAMPLE 1

o-Cyanobenzyl monate A

A solution containing sodium monate A (1.1 g, 3 mmol), o-cyanobenzyl bromide (0.6 g, 3 mmol), and dimethyl formamide (35 ml) was stirred at 20°C for 21 h and then evaporated *in vacuo*. The residue was taken up in ethyl acetate, washed with aqueous sodium bicarbonate and then brine, dried (magnesium sulphate) and evaporated *in vacuo*. The resulting residue was purified by chromatography (2 to 6% methanol in dichloromethane, 20 g silicagel) to give o-cyanobenzyl monate A as a colourless oil (0.72 g, 52%,

i.r. spectrum: $\nu_{max}$ ($CHCl_3$)

3400, 2220, 1710, 1640, 1600 $cm^{-1}$;

uv spectrum: $\lambda_{max}$ (EtOH)

227 nm ($\epsilon_m$ = 20,378);

$^1H$ nmr: $\delta_H$ ($CDCl_3$)

7.28-7.70 (4H, m, aryl), 5.83 (1H, s, H2), 5.30 (2H, s, $C\underline{H}_2C_6H_4$), 2.23 (3H, s, $CH_3$-15), 1.21 (3H, d, $CH_3$-14), 0.93 (3H, d, $CH_3$-17);

$^{13}C$ nmr: $\delta_C$ ($CDCl_3$)

165.4 (C1), 158.8 (C3), 139.3 (C1"), 132.6 132.5, 128.8, 128.1 (C3", 4", 5", 6"), 116.5 (CN), 115.9 (C2), 111.2 (C2"), 74.4 (C5), 70.1 (C13), 69.8 (C7), 68.3 (C6), 64.9 (C16), 62.4 (C1'), 60.4 (C11), 55.2 (C10), 42.4 (C4), 42.1 (C12), 39.2 (C8), 31.1 (C9), 20.1 (C14), 18.8 (C15), 11.9 (C17);

mass spectrum: *m/e* (relative intensity)

459 ($M^+$, 0.4%), 311 (5), 244 (6), 227 (36), 141 (22), 134 (35), 116 (100) (Found $M^+$ = 459.2256, calculated $C_{25}H_{33}NO_7$, 459.2255).

EXAMPLE 2

m-Cyanobenzyl monate A

A solution containing sodium monate A (3.3 g, 9 mmol), m-cyanobenzyl bromide (1.8 g, 9 mmol), and dimethyl formamide (100 ml) was stirred at 20°C for 16 h and then evaporated *in vacuo*. The residue was taken up in ethyl acetate, which was washed with aqueous sodium bicarbonate and then brine dried (magnesium sulphate), and evaporated *in vacuo*. The resulting residue was purified by chromatography (0 to 4% methanol in chloroform, 30 g silica-gel) to give m-cyanobenzyl monate A as a colourless oil, (3.7 g, 90%);

i.r. spectrum: $\nu_{max}$ (film)

3450, 2230, 1715, 1645 $cm^{-1}$;

uv spectrum: $\lambda_{max}$ (EtOH)

225 nm ($\varepsilon_m$ = 22,700);

$^1H$ nmr: $\delta_H$ ($CDCl_3$)

7.4-7.6 (4H, m, aryl), 5.82 (1H, s, H2), 5.18 (2H, s, H1'), 2.21 (3H, s, $CH_3$-15), 1.20 (3H, d, $CH_3$-14), 0.92 (3H, d, $CH_3$-17);

$^{13}C$ nmr: $\delta_C$ ($CDCl_3$)

165.9 (C1), 158.9 (C3), 138.1 (C1"), 132.3 (C6"), 131.6 (C2"), 131.4 (C5"), 129.3 (C4"), 118.5 (CN), 116.6 (C2), 112.6 (C3"), 74.9 (C5), 71.2 (C13), 70.3 (C7), 68.9 (C6), 65.4 (C16), 64.0 (C1'), 61.2 (C11), 55.5 (C10), 43.0 (C4), 42.8 (C12), 39.6 (C8), 31.6 (C9), 20.7 (C14), 19.3 (C15), 12.6 (C17);

mass spectrum: *m/e* (relative intensity)

459 ($M^+$, 1%), 397 (6), 311 (6), 227 (44), 116 (100) (Found $M^+$ = 459.2266. Calculated for $C_{25}H_{33}NO_7$, 459.2256).

EXAMPLE 3

p-Cyanobenzyl monate A

A solution containing sodium monate A (3.3 g, 9 mmol), p-cyanobenzyl bromide (1.8 g, 9 mmol), and dimethyl formamide (100 ml) was stirred at 80°C for 16 h and then evaporated *in vacuo*. The resulting residue was purified by chromatography (0 to 4% methanol in chloroform, 30 g silicagel) to give p-cyanobenzyl monate A as a colourless oil (4.0 g, 97%);

i.r. spectrum: $\nu_{max}$ (film)

3450, 2230, 1715, 1645 $cm^{-1}$;

uv spectrum: $\lambda_{max}$ (EtOH)

231 nm ($\varepsilon_m$ = 31,800);

$^1H$ nmr: $\delta_H$ ($CDCl_3$)

7.5-7.7 (4H, ABq, aryl), 5.85 (1H, s, H2), 5.18 (2H, s, H1'), 2.26 (3H, s, $CH_3$-15), 1.23 (3H, d, $CH_3$-14), 0.96 (3H, d, $CH_3$-17);

$^{13}C$ nmr: $\delta_C$ ($CDCl_3$)

165.9 (C1), 158.9 (C3), 141.9 (C1"), 132.3 (C3"), 128.3 (C2"), 118.6 (CN), 116.6 (C2), 111.7 (C4"), 74.9 (C5), 71.3 (C13), 70.3 (C7), 68.9 (C6), 65.4 (C16), 64.3 (C1'), 61.2 (C11), 55.5 (C10), 43.0 (C4), 42.8 (C12), 39.6 (C8), 31.6 (C9), 20.8 (C14), 19.3 (C15), 12.6 (C17);

mass spectrum: *m/e* (relative intensity)

459 ($M^+$, 1%), 441 (1), 423 (1), 227 (30), 116 (100) (Found $M^+$ = 459.2250, $C_{25}H_{33}NO_7$ requires 459.2256).

EXAMPLE 4

1-(p-Cyanophenyl)ethyl monate A

A solution containing sodium monate A (2.00 g, 5.4 mmol), p-(1-bromoethyl)benzonitrile (6 mmol), and dimethyl formamide (50 ml) was stirred for 16 h at 20°C and then evaporated *in vacuo*. The residue was diluted with ethyl acetate, washed with aqueous sodium bicarbonate and then brine, dried (magnesium sulphate), evaporated *in vacuo*, and the resulting residue purified by chromatography (0 to 4% methanol in chloroform, 20 g silicagel) to give 1-(p-cyanophenyl)ethyl monate A as a colourless oil, (0.78 g, 31%);

i.r. spectrum: $\nu_{max}$ (film)

3450, 2230, 1710, 1640, 1610 $cm^{-1}$;

uv spectrum: $\lambda_{max}$ (EtOH)

230 nm ($\varepsilon_m$ = 25, 600);

$^1H$ nmr: $\delta_H$ ($CDCl_3$)

7.54 (4H, ABq, aryl), 5.9 (1H, q, H1'), 5.82 (1H, s, H2), 2.17 (3H, s, $CH_3$-15), 1.51 (3H, d, H2'), 1.18 (3H, d, $CH_3$-14), 0.90 (3H, d, $CH_3$-17);

$^{13}C$ nmr: $\delta_C$ ($CDCl_3$)

165.5 (C1), 158.4 (C3), 147.6 and 147.7 (C1"), 132.4 (C3", 5"), 126.7 (C2", 6"), 118.6 (CN), 117.0 (C2), 111.4 (C4"), 74.9 (C5), 71.3 (C13), 70.6 (C1'), 70.3 (C7), 68.9 (C6), 65.4 (C16), 61.2 (C11), 55.5 (C10), 43.0 (C12), 42.8 (C4), 39.6 (C8), 31.6 (C9), 22.2 (C2'), 20.8 (C14), 19.2 and 19.3 (C15), 12.6 (C17);

Mass Spectrum: m/e (relative intensity)

473 ($M^+$, 1%), 455 (1), 227 (46), 130 (100) (Found $M^+$ = 473.2418, $C_{26}H_{35}NO_7$ requires 473.2423).

EXAMPLE 5

m-Cyanobenzyl monate C

Monic acid C (0.32 g, 1 mmol) was taken up in methanol (10 ml) and sodium bicarbonate (0.08 g, 1 mmol) added and stirred for ½ h. After evaporation *in vacuo*, m-cyanobenzyl bromide (0.2 g, 1 mmol) and dimethyl formamide (15 mL) were added and stirred for 21 h at 20°C, and then evaporated *in vacuo*. The residue was taken up in ethyl acetate, washed with aqueous sodium bicarbonate, then brine, dried (magnesium sulphate), and evaporated *in vacuo*. The resulting residue was purified by chromatography (10 g silica gel, 0 to 4% methanol in dichloromethane) to yield m-cyanobenzyl monate C as a colourless oil (227 mg, 51%);

i.r. spectrum: $\nu_{max}$ ($CHCl_3$)

3400, 2240, 1710, 1640 $cm^{-1}$;

uv spectrum: $\lambda_{max}$ (EtOH)

225 nm ($\varepsilon_m$ 29,072);

$^1H$ nmr: $\delta_H$ ($CDCl_3$)

7.68 (1H, s, H2"), 7.62 (2H, d, H4",6"), 7.48 (1H, t, H5"), 5.85 (1H, s, H2), 5.55-5.35 (2H, m, CH=CH), 5.14 (2H, s, $CH_2$-1'), 2.24 (3H, s, $CH_3$-15), 1.17 (3H, d, $CH_3$-14), 0.98 (3H, d, $CH_3$-17);

$^{13}C$ nmr: $\delta_C$ ($CDCl_3$)

166.0 (C1), 159.1 (C3), 138.3 (C1"), 134.4, 129.4 (C10,11), 132.3 (C6"), 131.6 (C2"), 131.4 (C5"), 129.2 (C4"), 118.5 (CN), 116.6 (C2), 112.7 (C3"), 74.9 (C5), 71.3 (C13), 70.4 (C7), 68.9 (C6), 64.9 (C16), 64.0 (C1'), 44.5 (C4), 43.2 (C12), 42.1 (C8), 32.4 (C9), 20.4 (C14), 19.4 (C15), 16.6 (C17).

EXAMPLE 6

3,5-Dicyanobenzyl monate A

A mixture of 3,5-dicyanotoluene (0.5 g, 3.5 mmol), bromine (0.2 ml, 3.5 mmol) and nitrobenzene (2 ml) was heated at 200°C for 1½ h and then cooled. The resulting solution was treated with sodium monate A (1.1 g, 3 mmol) and dimethyl formamide (30 ml) for 3 days at 20°C, and then evaporated *in vacuo*. The residue was dissolved in ethyl acetate, washed with aqueous sodium bicarbonate, dried (magnesium sulphate) and evaporated *in vacuo*. The resulting residue was purified by chromatography (on silicagel eluting with 0 to 7% methanol in dichloromethane) to give 3,5-dicyanobenzyl monate A as a colourless oil (0.13 g, 99%);

i.r. spectrum: $\nu_{max}$ (film)

3460, 2240, 1720, 1650, 755 $cm^{-1}$;

uv spectrum: $\lambda_{max}$ (EtOH)

223 nm ($\varepsilon_m$ 26,000);

$^1H$ nmr: $\delta_H$ ($CDCl_3$)

7.90 (3H, s, aryl), 5.85 (1H, s, H2), 5.20 (2H, s, H1'), 2.20 (3H, s, $CH_3$-15), 1.20 (3H, d, $CH_3$-14), 0.90 (3H, d, $CH_3$-17);

$^{13}C$ nmr: $\delta_C$ ($CDCl_3$)

165.6 (C1), 160.2 (C3), 140.4 (C1"), 135.0 (C2",6"), 134.6 (C4"), 116.4 (C3", 5"), 116.1 (C2), 114.5 (CN), 75.0 (C5), 71.3 (C13), 70.5 (C7), 69.1 (C6), 65.5 (C5), 62.9 (C1'), 61.2 (C11), 55.5 (C10), 43.1 (C12), 42.9 (C4), 39.8 (C8), 31.7 (C9), 20.8 (C14), 19.5 (C15), 12.6 (C17);

Mass Spectrum: m/e (relative intensity)

484 ($M^+$, 1), 422 (9), 227 (81), 141 (100) (Found: 484.2213. $C_{26}H_{32}N_2O_7$ requires 484.2208).

## Biological Data

### a) Anti-mycoplasmal Activity

Table 1 shows the *in vitro* MIC values (μg/ml) of the compounds of the Examples against a number of mycoplasma organisms. The values were determined in Friis broth solidified with 0.9% agarose. The inoculum was $10^3$ to $10^5$ CFU and the MIC's were recorded after 6 days incubation at 37°C.

TABLE 1

| ORGANISMS | EXAMPLE NO. - MIC (μg/ml) | | | | | |
|---|---|---|---|---|---|---|
| | 1 | 2 | 3 | 4 | 5 | 6 |
| M. suipneumoniae NB12 | 0.5 | 0.05 | 0.25 | 1.0 | 0.5 | 0.1 |
| M. suipneumoniae JF 435 | 1.0 | 0.1 | 0.25 | 1.0 | 1.0 | 0.1 |
| M. suipneumoniae HK(2) | 1.0 | 0.1 | 0.25 | 2.5 | 1.0 | 0.1 |
| M. suipneumoniae Str. 11 | 1.0 | 0.05 | 0.1 | 1.0 | 0.5 | 0.05 |
| M. suipneumoniae J2206/183b | 1.0 | 0.05 | 0.25 | 2.5 | 1.0 | 0.1 |
| M. suipneumoniae MS 16 | 0.5 | 0.05 | 0.1 | 1.0 | 0.5 | 0.05 |
| M. suipneumoniae PW/C/210 | 0.5 | 0.05 | 0.1 | 1.0 | 0.5 | 0.05 |
| M. suipneumoniae LABER | 0.5 | 0.05 | 0.1 | 1.0 | 0.5 | 0.05 |
| M. suipneumoniae UCD 1 | 1.0 | 0.1 | 0.25 | 1.0 | 1.0 | 0.1 |
| M. suipneumoniae TAM 6N | 2.5 | 0.1 | 0.25 | 2.5 | 1.0 | 0.1 |
| M. suipneumoniae ATCC 25095 | 0.5 | 0.05 | 0.25 | 1.0 | 0.5 | 0.05 |
| M. suipneumoniae NCTC 10110 | 1.0 | 0.05 | 0.25 | 2.5 | 1.0 | 0.1 |
| M. hyorhinis ATCC 23234 | 0.5 | 0.025 | 0.1 | 0.5 | 0.5 | 0.05 |
| M. hyorhinis ATCC 25021 | 0.5 | 0.025 | 0.1 | 0.5 | 0.5 | 0.05 |
| M. hyosynoviae ATCC 25591 | ≤0.01 | 0.05 | <0.01 | 0.1 | 0.5 | 0.1 |
| M. bovis NCTC 10131 | | ≤0.01 | <0.01 | ≤0.01 | 0.05 | 0.025 |
| M. bovigenitalium ATCC 14173 | ≤0.01 | ≤0.01 | ≤0.01 | 0.025 | 0.1 | 0.025 |
| M. dispar NCTC 10125 | 0.25 | 0.025 | 0.05 | 0.5 | 0.1 | 0.05 |
| M. gallisepticum S6 | 2.5 | 2.5 | 0.25 | 10 | 10.0 | 2.5 |
| M. pneumoniae ATCC 15492 | 2.5 | 0.5 | 0.25 | 5.0 | 5.0 | 2.5 |

b) Veterinary Bacteria

Table 2 shows the MIC values (μg/ml) of the compounds of the Examples against a number of organisms important in veterinary infections. The values were determined using a two fold serial dilutions in Diagnostic Sensitivity Test Agar with an inoculum of $10^4$ organisms and incubation for 18 hours at $37^oC$.

TABLE 2

| ORGANISMS | EXAMPLE NO. - MIC (μg/ml) | | | | | |
|---|---|---|---|---|---|---|
| | 1 | 2 | 3 | 4 | 5 | 6 |
| E. Coli NCTC 10418 | >80 | 80 | 80 | >80 | > 80 | > 80 |
| E. Coli El | >80 | 80 | 80 | >80 | > 80 | > 80 |
| S. dublin S7 | >80 | 80 | >80 | >80 | > 80 | > 80 |
| S. typhimurium S18 | >80 | 80 | 80 | >80 | > 80 | > 80 |
| Bord. bronchiseptica BO8 | >80 | 20 | 20 | 80 | > 80 | 80 |
| Bord. bronchiseptica BO9 | 10 | 10 | 10 | 20 | 20 | 40 |
| Past. multocida PA1 | 1.25 | .625 | .625 | 10 | 10 | 5 |
| Past. multocida PA2 | .156 | .156 | .156 | <.019 | 5 | 0.625 |
| Past. haemolytica PA5 | 10 | 2.5 | 2.5 | 40 | 40 | 10 |
| Erysipelothrix rhusiopathiae NCTC 8163 | 80 | 20 | 40 | 40 | 80 | 80 |
| Corynebacterium pyogenes CY1 | >80 | >80 | >80 | >80 | > 80 | > 80 |
| Staph. aureus B4 | 1.25 | 1.25 | .625 | 10 | 2.5 | 5 |
| Staph. aureus 152 | 1.25 | .625 | .312 | 5 | 2.5 | 5 |
| Staph. aureus Oxford | 1.25 | .625 | .312 | 2.5 | 2.5 | 5 |
| Strep. suis (group D) SPS11 | 10 | 2.5 | 2.5 | 20 | > 80 | 40 |
| Strep. uberis SPU1 | .156 | .312 | .07 | 1.25 | 0.156 | 0.312 |
| Strep. dysgalactiae SPD1 | .312 | .312 | .156 | 1.25 | 0.156 | 0.625 |
| Strep. agalactiae SPA1 | 2.5 | .625 | 1.25 | 10 | 2.5 | 0.625 |
| B. subtilis ATCC 6633 | | | | | | NG |

c) Human Bacteria

Table 3 shows the MIC values (μg/ml) of the compounds of the Examples against a number of organisms important in human infections. The values were determined by serial dilutions in nutrient agar with 5% chocolated horse blood after incubations for 18 hours at 37°C.

TABLE 3

| ORGANISMS | EXAMPLE NO. - MIC µg/ml) | | | | | |
|---|---|---|---|---|---|---|
| | 1 | 2 | 3 | 4 | 5 | 6 |
| E. coli NCTC 10418 | >100 | 50 | 50 | >100 | >100 | > 100 |
| E. coli ESS | 1.0 | 0.5 | 0.25 | 2.5 | 5 | 5.0 |
| P. mirabilis 889 | >100 | 100 | 50 | >100 | >100 | 100 |
| K. aerogenes A | >100 | 100 | 50 | >100 | >100 | > 100 |
| Ps. aeruginosa NCTC 10662 | >100 | >100 | >100 | >100 | >100 | > 100 |
| Pasteurella multocida 1633 | 1.0 | 1.0 | 0.5 | 10 | 10 | 2.5 |
| Haemophilus influenzae Q1 | 0.25 | 0.1 | 0.1 | 0.5 | - | 0.5 |
| Haemophilus influenzae Wy21 | 0.25 | 0.1 | - | 0.5 | 2.5 | - |
| Neisseria catarrhalis 1502 | 0.5 | 0.25 | 0.1 | 0.5 | 2.5 | 0.5 |
| Bacillus subtilis 6633 | 2.5 | 0.5 | 0.1 | 1.0 | 2.5 | 2.5 |
| Corynebacterium xerosis 9755 | >100 | >100 | >100 | >100 | >100 | > 100 |
| Sarcina lutea 8340 | >100 | >100 | >100 | >100 | >100 | > 100 |
| Staph. aureus Oxford | 1.0 | 1.0 | 0.25 | 2.5 | 2.5 | 2.5 |
| Staph. aureus Russell | 1.0 | 2.5 | 0.5 | 5.0 | 2.5 | 5.0 |
| Staph. aureus W2827 | 2.5 | 1.0 | 0.5 | 5.0 | 2.5 | 5.0 |
| Strep. faecalis I | >100 | 100 | 100 | >100 | >100 | 100 |
| Strep. pyogenes R80/421-A | 0.5 | - | 0.1 | - | 2.5 | 1.0 |
| Strep. 2788-B | 5.0 | 1.0 | 0.5 | 5.0 | 5.0 | - |
| Strep. 641848-C | 1.0 | 1.0 | 0.25 | 2.5 | 2.5 | 1.0 |
| Strep. pneumoniae CN33 | 1.0 | 0.5 | 0.25 | 2.5 | - | - |

d) Swine Dysentry

The activity of the compounds of Examples 2 and 3 against various strains of Treponema hyodysenterial, associated with swine dysentry, were determined.

MIC values (µg/ml) of the compounds were determined by incorporating the compounds in horse blood agar, inoculating (multi-point) with 0.001 ml of 3 day growth from base of blood slope (+ 1 ml foetal calf serum) and incubating at 37°C for 3 days under aneorobic conditions (Gas Pak 150). The MIC values were taken as the lowest concentrations to inhibit β-haemolysis.

The results are as follows:

| Strain No. | Example No. | |
|---|---|---|
| | 2 | 3 |
| S77/2 | 10 | 10 |
| S77/10 | 10 | 10 |
| P23 | 10 | 40 |
| Navis | 10 | 10 |
| B10 | 10 | 20 |
| $TH_3$ | - | 5 |
| S73/2 | - | 5 |

CLAIMS

1. A compound of formula (II):

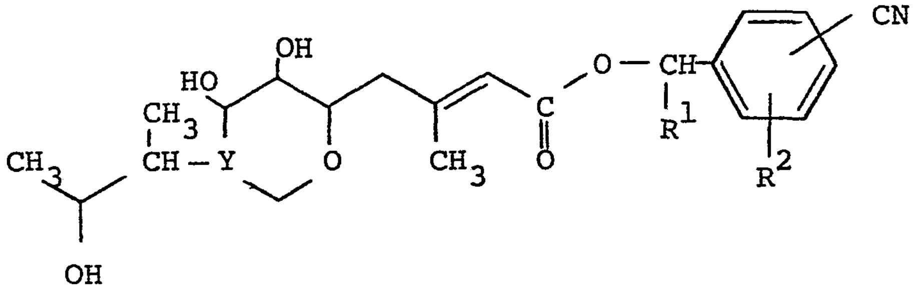

(II)

wherein Y is selected from $-CH{=}CH-CH_2-CH-$,
$-CH-CH-CH_2-CH-$ and $-CH-CH-CH_2-C(OH)-$;
 \O/                 \O/
$R^1$ represents hydrogen or a $C_{1-4}$ alkyl group and $R^2$ is hydrogen or a cyano group.

2. A compound according to claim 1, in which $R^2$ represents hydrogen.

3. A compound according to claim 1 or claim 2, in which $R^1$ represents hydrogen or a methyl group.

4. A compound selected from o-cyanobenzyl monate A, m-cyanobenzyl monate A, p-cyanobenzyl monate A, 1-(p-cyanobenzyl)ethyl monate A, m-cyanobenzyl monate C and 3,5-dicyanobenzyl monate A.

5. A process for producing a compound of formula (II) as defined in claim 1, which comprises reacting a cyanobenzyl compound of formula (III):

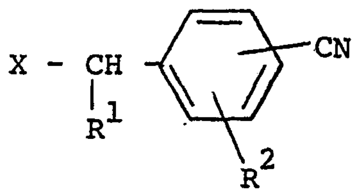

(III)

in which X is a good leaving group and $R^1$ and $R^2$ are as defined in formula (II), with a salt of an acid of formula (I):

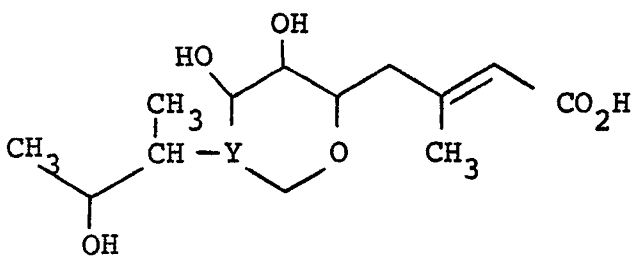

(I)

wherein Y is as defined in formula (II).

6. A process according to claim 5, in which X represents a halogen atom or an alkyl or aryl sulphonate.

7. A pharmaceutical or veterinary composition comprising a compound of formula (II) as defined in claim 1 in association with a pharmaceutically or veterinarily acceptable carrier therefor.

8. A pharmaceutical or veterinary composition according to claim 7, in unit dosage form.

9. A compound of formula (II) as defined in claim 1, for use in the treatment of the human or animal body.

0052437

European Patent Office

# EUROPEAN SEARCH REPORT

Application number
EP 81 30 4988

## DOCUMENTS CONSIDERED TO BE RELEVANT

| Category | Citation of document with indication, where appropriate, of relevant passages | Relevant to claim |
|---|---|---|
| P | GB - A - 1 587 059 (BEECHAM)<br>* pages 1-6, 15; examples 18,22-24 *<br>--------- | 1-3,5-8 |

CLASSIFICATION OF THE APPLICATION (Int. Cl. 3)

C 07 D 407/06
309/10
A 61 K 31/35

TECHNICAL FIELDS SEARCHED (Int.Cl. 3)

A 61 K 31/00
C 07 D 407/00
309/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 17-02-1982 | FRANCOIS |

EPO Form 1503.1 06.78